(19) Europäisches Patentamt
European Patent Office
Office européen des brevets

(11) Veröffentlichungsnummer: **0 039 813**
**B1**

(12)
# EUROPÄISCHE PATENTSCHRIFT

(45) Veröffentlichungstag der Patentschrift: **20.07.83**

(51) Int. Cl.³: **C 07 C 119/06** // C07C103/375

(21) Anmeldenummer: **81103131.9**

(22) Anmeldetag: **27.04.81**

(54) **Fluorierte Azomethine, Verfahren zu ihrer Herstellung und ihre Verwendung als Zwischenprodukte.**

(30) Priorität: **10.05.80 DE 3018020**

(43) Veröffentlichungstag der Anmeldung: **18.11.81 Patentblatt 81/46**

(45) Bekanntmachung des Hinweises auf die Patenterteilung: **20.07.83 Patentblatt 83/29**

(84) Benannte Vertragsstaaten: **AT BE CH DE FR GB IT LI NL**

(56) Entgegenhaltungen:
**THE JOURNAL OF ORGANIC CHEMISTRY, Band 35, Nr. 2, Februar 1970, Seiten 340-344, Washington D.C., USA. J. HINE et al.: "Polar effects on the formation of imines from isobutyraldehyde and primary aliphatic amines"**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Baasner, Bernd, Dr., Kalkstrasse 132, D-5090 Leverkusen 1 (DE)**
Erfinder: **Klauke, Erich, Dr., Eichendorffweg 8, D-5068 Odenthal (DE)**
Erfinder: **Hagemann, Hermann, Dr., Roggendorfstrasse 55, D-5000 Köln 80 (DE)**
Erfinder: **Kühle, Engelbert, Dr., von-Bodelschwingh-Strasse 42, D-5060 Bergisch-Gladbach 2 (DE)**

**EP 0 039 813 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Fluorierte Azomethine, Verfahren zu ihrer Herstellung und ihre Verwendung als Zwischenprodukte

Die vorliegende Erfindung betrifft neue fluorierte Azomethine, ein Verfahren zu ihrer Herstellung sowie ihre Verwendung als Zwischenprodukte zur Synthese von Verbindungen, welche eine antagonistische Wirkung auf bestimmte Herbizide ausüben.

Es ist bereits bekannt geworden, bestimmte Azomethine durch Umsetzung von Aminen mit Carbonylverbindungen herzustellen [vgl. J. Amer. Chem. Soc. 66, 82 (1944)].

Von den entsprechenden fluorierten Azomethinen wurde bisher nur das N-Isobutyliden-2,2,2-trifluorethyl-amin beschrieben [vgl. J. Org. Chem. 35, (1970), 340–344]. Eine Verwendung dieses Stoffes für irgendeinen praktischen Zweck wurde allerdings noch nicht offenbart.

Weiterhin ist bereits bekannt geworden, dass N,N-Di-n-propyl-dichloracetamid geeignet ist als Antidot («Safener», «Gegenmittel»), um die Kulturpflanzenverträglichkeit von herbizid wirksamen Thiolcarbamaten oder Acetaniliden zu verbessern (vgl. DE-OS 2 218 097). Die Wirksamkeit dieses Stoffes als Antidot ist allerdings nicht immer ganz befriedigend.

Unter «Antidots» sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.

Es wurden nun neue fluorierte Azomethine der Formel

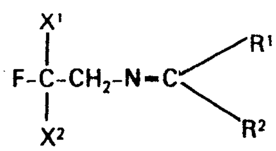

(I)

in welcher
$X^1$ für Wasserstoff, Fluor oder Chlor steht,
$X^2$ für Wasserstoff, Fluor oder Chlor steht,
$R^1$ für Wasserstoff, Methyl, Ethyl, n-Propyl oder n-Butyl steht und
$R^2$ für Wasserstoff, Methyl oder Ethyl steht,
gefunden.

Weiterhin wurde gefunden, dass man die neuen fluorierten Azomethine der Formel (I) erhält, wenn man fluorierte Amine der Formel

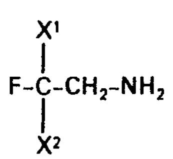

(II)

in welcher
$X^1$ und $X^2$ die oben angegebene Bedeutung haben, mit Carbonylverbindungen der Formel

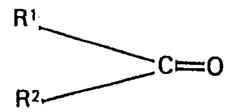

(III)

in welcher
$R^1$ und $R^2$ die oben angegebene Bedeutung haben, gegebenenfalls in Gegenwart eines Verdünnungsmittels, umsetzt.

Ausserdem wurde gefunden, dass die neuen fluorierten Azomethine der Formel (I) als Zwischenprodukte zur Herstellung von fluorierten Halogenacetamiden der Formel

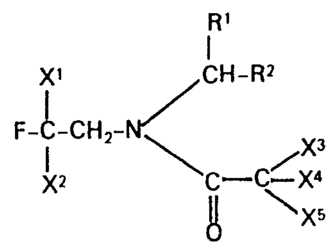

(IV)

in welcher
$X^1$, $X^2$, $R^1$ und $R^2$ die oben angegebene Bedeutung haben,
$X^3$ für Wasserstoff, Fluor oder Chlor steht,
$X^4$ für Wasserstoff, Fluor oder Chlor steht und
$X^5$ für Fluor oder Chlor steht,
eingesetzt werden können. Die fluorierten Halogenacetamide der Formel (IV) üben eine antagonistische Wirkung auf herbizid wirksame Thiolcarbamate und Acetanilide aus.

Überraschenderweise sind die fluorierten Halogenacetamide der Formel (IV), die sich aus den erfindungsgemässen fluorierten Azomethinen der Formel (I) durch Hydrierung und anschliessende Umsetzung der dabei entstehenden fluorierten Amine mit Halogenessigsäurechloriden herstellen lassen, besser zur Erhöhung der Kulturpflanzenverträglichkeit von herbizid wirksamen Thiolcarbamaten oder Acetaniliden geignet als das aus dem Stand der Technik bekannte N,N-Di-n-propyl-dichloracetamid, welches ein hochaktiver Wirkstoff gleicher Wirkungsart ist. Die erfindungsgemässen Stoffe stellen somit als Zwischenprodukte zur Synthese von Antidots eine wertvolle Bereicherung der Technik dar.

Verwendet man 2,2-Difluorethylamin und Acetaldehyd als Ausgangsstoffe, so lässt sich der Verlauf des erfindungsgemässen Verfahrens durch das folgende Formelschema wiedergeben:

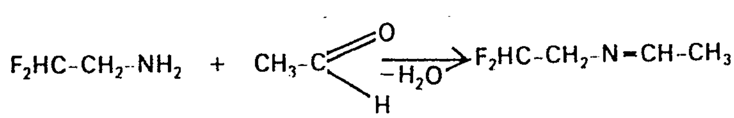

Die bei dem erfindungsgemässen Verfahren als Ausgangsstoffe benötigten fluorierten Amine sind durch die Formel (II) allgemein definiert. In dieser Formel haben $X^1$ und $X^2$ diejenigen Bedeutungen, die bereits im Zusammenhang mit der Beschreibung der erfindungsgemässen Stoffe der Formel (I) für diese Reste genannt wurden.

Die fluorierten Amine der Formel (II) sind bekannt oder lassen sich nach im Prinzip bekannten Verfahren herstellen [vgl. J. Org. Chem. 24 (1959), 1256–1259].

Die bei dem erfindungsgemässen Verfahren weiterhin als Ausgangsstoffe benötigten Carbonylverbindungen sind durch die Formel (III) allgemein definiert. In dieser Formel stehen $R^1$ und $R^2$ für diejenigen Reste, die bereits im Zusammenhang mit der Beschreibung der erfindungsgemässen Stoffe der Formel (I) für $R^1$ und $R^2$ genannt wurden.

Als Beispiele für Carbonylverbindungen der Formel (III) seien im einzelnen genannt:

Formaldehyd
Acetaldehyd
Propionaldehyd
Butyraldehyd
Aceton
Diethylketon

Die Carbonylverbindungen der Formel (III) sind bekannt.

Die Umsetzung nach dem erfindungsgemässen Verfahren wird gegebenenfalls in Gegenwart eines Verdünnungsmittels durchgeführt. Als Verdünnungsmittel kommen dabei inerte organische Solventien in Frage. Vorzugsweise verwendbar sind gegebenenfalls halogenierte aliphatische und cycloaliphatische Kohlenwasserstoffe, wie Pentan, Hexan, Cyclohexan, Chloroform und Tetrachlorkohlenstoff, ferner gegebenenfalls halogenierte aromatische Kohlenwasserstoffe, wie Benzol, Xylol, Toluol und Chlorbenzol, und ausserdem Ether, wie Diethylether, Tetrahydrofuran und Dioxan.

Die Reaktionstemperaturen können bei dem erfindungsgemässen Verfahren innerhalb eines bestimmten Bereiches variiert werden. Im allgemeinen arbeitet man bei Temperaturen zwischen −20°C und +60°C, vorzugsweise zwischen 0°C und +40°C.

Bei der Durchführung des erfindungsgemässen Verfahrens setzt man auf 1 Mol an fluoriertem Amin der Formel (II) 1 bis 1,2 Mol an Carbonylverbindung der Formel (III) ein. Die Aufarbeitung erfolgt nach üblichen Methoden. Im allgemeinen verfährt man in der Weise, dass man nach beendeter Umsetzung festes Alkalihydroxid, vorzugsweise Natrium- oder Kaliumhydroxid, hinzufügt, dann die sich daraufhin abscheidende organische Phase abtrennt, trocknet und destilliert.

Die erfindungsgemässen fluorierten Azomethine der Formel (I) eignen sich als Zwischenprodukte zur Synthese von fluorierten Halogenacetamiden der Formel (IV), welche eine antagonistische Wirkung auf bestimmte herbizid wirksame Thiolcarbamate und Acetanilide ausüben. Die fluorierten Halogenacetamide der Formel (IV) lassen sich herstellen, indem man fluorierte Azomethine der Formel

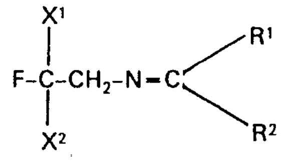

(I)

in welcher
$X^1$, $X^2$, $R^1$ und $R^2$ die oben angegebene Bedeutung haben, mit Wasserstoff unter einem Druck von 3 bis 15 bar in Gegenwart eines Katalysators, wie Platin auf Kohle, Palladium auf Kohle oder Raney-Nickel, sowie in Gegenwart eines Verdünnungsmittels, zum Beispiel eines Alkohols wie Methanol oder Ethanol, oder eines Ethers wie Dioxan, bei Temperaturen zwischen 0°C und 60°C, vorzugsweise zwischen 10°C und 50°C hydriert und dann die dabei entstehenden fluorierten Amine der Formel

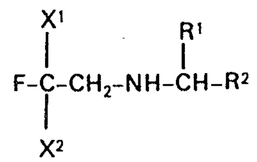

(V)

in welcher
$X^1$, $X^2$, $R^1$ und $R^2$ die oben angegebene Bedeutung haben, mit Halogenessigsäurechloriden der Formel

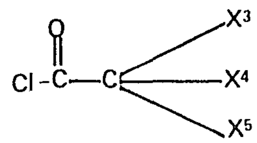

(VI)

in welcher
$X^3$, $X^4$ und $X^5$ die oben angegebene Bedeutung haben, gegebenenfalls in Gegenwart eines Säurebindemittels, wie Triethylamin, sowie gegebenenfalls in Gegenwart eines Verdünnungsmittels, wie Toluol, bei Temperaturen zwischen 0°C und 80°C umsetzt.

So lässt sich beispielsweise das Dichloressigsäure-N-(2,2,2-trifluorethyl)-N-ethyl-amid der Formel

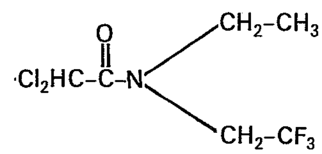

herstellen, indem man in einer ersten Stufe Ethyliden-2,2,2-trifluorethylamin in Gegenwart von Ethanol sowie in Gegenwart von Platin auf Kohle als Katalysator unter einem Wasserstoffdruck

von 10 bar hydriert, und das dabei entstehende N-(2,2,2-Trifluorethyl)-N-ethylamin dann in einer zweiten Stufe mit Dichloracetylchlorid in Gegenwart von Triethylamin als Säurebindemittel sowie in Gegenwart von Toluol als Verdünnungsmittel umsetzt. Diese Synthese lässt sich formelmässig wie folgt wiedergeben:

1. Stufe:

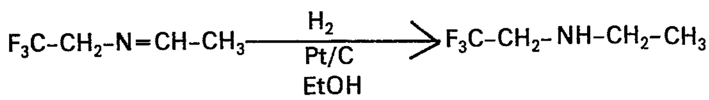


2. Stufe:

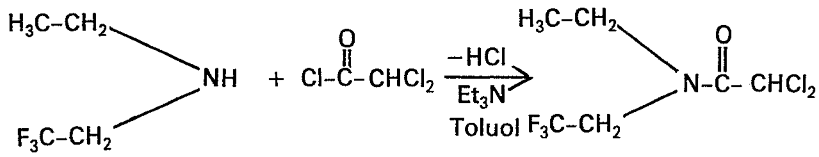


Die fluorierten Halogenacetamide der Formel (IV) sind geeignet, um die Kulturpflanzenverträglichkeit von bestimmten herbizid wirksamen Thiolcarbamaten und Acetaniliden zu verbessern. Vorzugsweise lassen sie sich als Antidots einsetzen bei Thiolcarbamaten der Formel

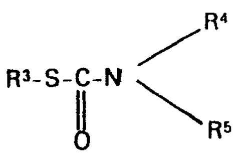

(VII)

in welcher
$R^3$ für niederes Alkyl, Benzyl, Chlorbenzyl oder Alkoxybenzyl steht,
$R^4$ und $R^5$ unabhängig voneinander für Alkyl mit 2 bis 4 Kohlenstoffatomen oder für Cyclohexyl stehen und ausserdem
$R^4$ und $R^5$ gemeinsam mit dem angrenzenden Stickstoffatom für einen fünf- bis siebengliedrigen heterocyclischen Ring stehen,
bzw. bei Acetaniliden der Formel

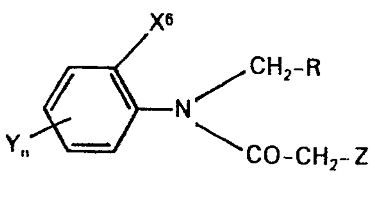

(VIII)

in welcher
R für einen gegebenenfalls substituierten über N gebundenen Azolrest steht
$X^6$ und Y gleich oder verschieden sind und für Alkyl stehen,
Z für Halogen steht und
n für 0, 1 oder 2 steht,
bzw. der Formeln

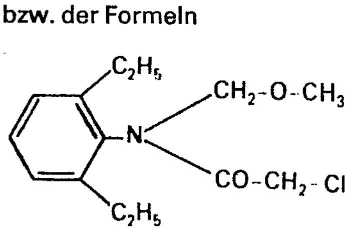

(IX)

und

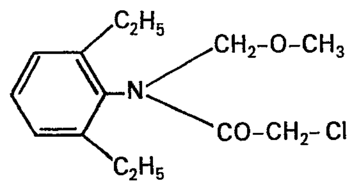

(IX)

Die gute antagonistische Wirkung der fluorierten Halogenacetamide der Formel (IV) geht aus dem nachfolgenden Beispiel hervor.

In diesem Beispiel werden die nachstehend angegebenen Stoffe als Test-Komponenten (Antidot bzw. Herbizid) eingesetzt:

(A) =

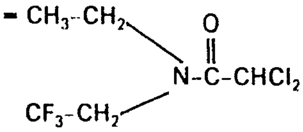

(Dichloressigsäure-N-(2,2,2-trifluorethyl)-N-ethyl-amid)

(B) =

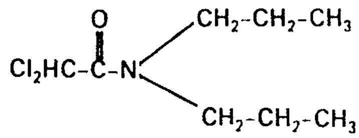

(N,N-Di-n-propyl-dichloracetamid)

(C) =

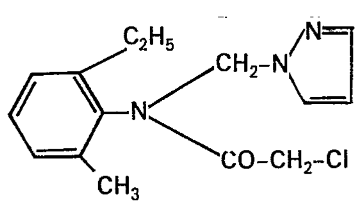

(2-Methyl-6-ethyl-N-(pyrazol-1-yl-methyl)-chloracetanilid)

Beispiel A
Pre-emergence-Test

Lösungsmittel: 5 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmässigerweie konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% keine Wirkung (wie unbehandelte Kontrolle)
100% totale Vernichtung

Eine Auswertung der Testergebnisse zeigt, dass das fluorierte Halogenacetamid (A) besser zur Erhöhung der Kulturpflanzenverträglichkeit des Herbizids (C) geeignet ist als die Vergleichskomponente (B).

Herstellungsbeispiele

Beispiel 1

$CF_3-CH_2-N=CH-CH_3$

Zu 99 g (1 Mol) 2,2,2-Trifluorethylamin werden innerhalb von 60 Minuten unter Eiskühlung 44 g (1 Mol) frisch destillierter Acetaldehyd zugetropft. Man lässt 1 Stunde nachrühren, gibt 15 g festes Kaliumhydroxid hinzu und trennt die Phasen. Die organische Phase wird über etwa 5 g festem Kaliumhydroxid getrocknet und anschliessend über eine kurze Kolonne destilliert. Nach einem Vorlauf (20 g; Kp: 18–42°C), der aus nicht umgesetztem Amin und Acetaldehyd besteht, erhält man 77,5 g (62% der Theorie) an Ethyliden-2,2,2-trifluorethyl-amin.

Kp=73–74°C
$n_D^{20} = 1,3415$

Nach der im Beispiel 1 angegebenen Methode werden auch die in den folgenden Beispielen formelmässig aufgeführten Verbindungen der Formel (I) erhalten.

Beispiel 2

$CF_3CH_2-N=CH-CH_2-CH_3$

Ausbeute: 64% der Theorie
Kp. = 93–95°C

Beispiel 3

$CF_3-CH_2-N=CH-CH_2-CH_2-CH_2$

Ausbeute: 72% der Theorie
Kp. = 116–118°C

Beispiel IV-1
Herstellung des Dichloressigsäure-N-(2,2,2-trifluormethyl)-N-ethyl-amids

a)

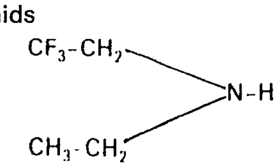

125 g (1 Mol) Ethyliden-2,2,2-trifluorethylamin werden in 250 ml absolutem Ethanol gelöst, mit 4 g Platin auf Kohle (5%ig) versetzt und bei 30°C unter einem Wasserstoffdruck von 10 bar 90 Minuten lang hydriert. Nach dem Abfiltrieren des Katalysators wird mit konzentrierter Salzsäure angesäuert und unter vermindertem Druck bis zur Trockne eingeengt. Das dabei anfallende Produkt wird mit 100 ml 50%iger wässriger Natronlauge versetzt und destilliert. Man erhält auf diese Weise 83 g (65% der Theorie) an 2,2,2-Trifluorethyl-N-ethylamin.

Kp. = 61–62°C
$n_D^{20} = 1,3335$

b)

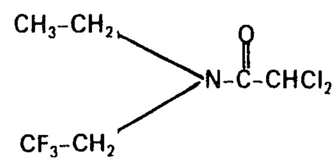

Zu einer Lösung von 12,7 g (0,1 Mol) 2,2,2-Trifluorethyl-N-ethylamin und 10,1 g (0,1 Mol) Triethylamin in 50 ml Toluol tropft man unter Eiskühlung eine Lösung von 14,7 g (0,1 Mol) Dichloracetylchlorid in 20 ml Toluol. Hierbei lässt man die Temperatur des Reaktionsgemisches auf 30°C ansteigen. Man rührt 30 Minuten nach, saugt dann vom ausgefallenen Feststoff ab und engt das Filtrat unter vermindertem Druck ein. Man erhält auf diese Weise 23,8 g (Ausbeute quantitativ) an Dichloressigsäure-N-(2,2,2-trifluorethyl)-N-ethyl-amid mit einem Brechungsindex von $n_D^{20} = 1,4472$.

**Patentansprüche**

1. Fluorierte Azomethine der Formel

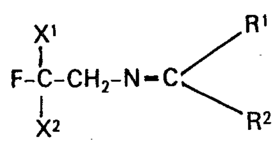

(I)

in welcher
$X^1$ für Wasserstoff, Fluor oder Chlor steht,
$X^2$ für Wasserstoff, Fluor oder Chlor steht,
$R^1$ für Wasserstoff, Methyl, Ethyl, n-Propyl oder n-Butyl steht und
$R^2$ für Wasserstoff, Methyl oder Ethyl steht.

2. Verfahren zur Herstellung von fluorierten Azomethinen der im Anspruch 1 angegebenen Formel (I), dadurch gekennzeichnet, dass man fluorierte Amine der Formel

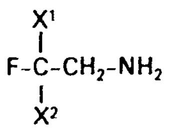
(II)

in welcher
$X^1$ und $X^2$ die im Anspruch 1 angegebene Bedeutung haben, mit Carbonylverbindungen der Formel

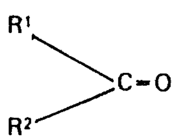
(III)

in welcher
$R^1$ und $R^2$ die im Anspruch 1 angegebene Bedeutung haben, gegebenenfalls in Gegenwart eines Verdünnungsmittels, umsetzt.

3. Verwendung von fluorierten Azomethinen der im Anspruch 1 angegebenen Formel (I) als Zwischenprodukte zur Synthese von fluorierten Halogenacetamiden der Formel

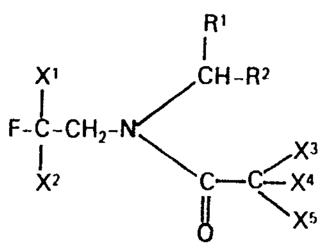
(IV)

in welcher
$X^1$, $X^2$, $R^1$ und $R^2$ die im Anspruch 1 angegebene Bedeutung haben,
$X^3$ für Wasserstoff, Fluor oder Chlor steht,
$X^4$ für Wasserstoff, Fluor oder Chlor steht und
$X^5$ für Fluor oder Chlor steht,
dadurch gekennzeichnet, dass man fluorierte Azomethine der Formel

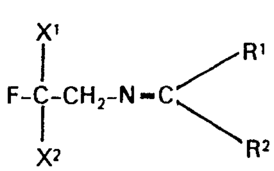
(I)

in welcher
$X^1$, $X^2$, $R^1$ und $R^2$ die oben angegebene Bedeutung haben, mit Wasserstoff unter einem Druck von 3 bis 15 bar in Gegenwart eines Katalysators sowie in Gegenwart eines Verdünnungsmittels bei Temperaturen zwischen 0°C und 60°C hydriert und die dabei entstehenden fluorierten Amine der Formel

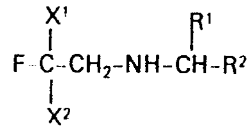
(V)

in welcher
$X^1$, $X^2$, und $R^1$, $R^2$ die oben angegebene Bedeutung haben, mit Halogenessigsäurechloriden der Formel

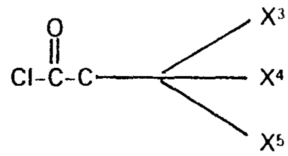
(VI)

in welcher
$X^3$, $X^4$ und $X^5$ die oben angegebene Bedeutung haben, gegebenenfalls in Gegenwart eines Säurebindemittels sowie gegebenenfalls in Gegenwart eines Verdünnungsmittels, bei Temperaturen zwischen 0°C und 80°C umsetzt.

## Claims

1. Fluorinated azomethines of the formula

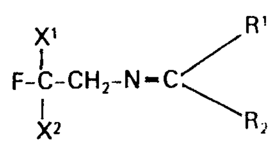
(I)

in which
$X^1$ represents hydrogen, fluorine or chlorine,
$X^2$ represents hydrogen, fluorine or chlorine,
$R^1$ represents hydrogen, methyl, ethyl, n-propyl or n-butyl and
$R^2$ represents hydrogen, methyl or ethyl.

2. Process for the preparation of fluorinated azomethines of the formula (I) given in Claim 1, characterised in that fluorinated amines of the formula

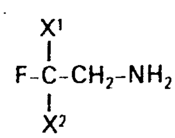
(II)

in which
$X^1$ and $X^2$ have the meaning given in Claim 1, are reacted with carbonyl compounds of the formula

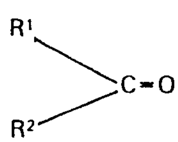
(III)

in which

$R^1$ and $R^2$ have the meaning given in Claim 1, if appropriate in the presence of a diluent.

3. Use of fluorinated azomethines of the formula (I) given in Claim 1 as intermediate products for the synthesis of fluorinated halogenoacetamides of the formula

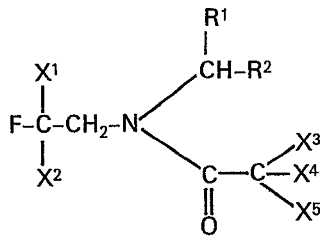

(IV)

in which
$X^1$, $X^2$, $R^1$ and $R^2$ have the meaning given in Claim 1,
$X^3$ represents hydrogen, fluorine or chlorine,
$X^4$ represents hydrogen, fluorine or chlorine and
$X^5$ represents fluorine or chlorine,
characterised in that fluorinated azomethines of the formula

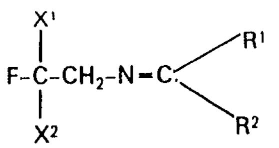

(I)

in which
$X^1$, $X^2$, $R^1$ and $R^2$ have the abovementioned meaning, are hydrogenated with hydrogen under a pressure of 3 to 15 bars, in the presence of a catalyst and in the presence of a diluent, at temperatures between 0°C and 60°C and the resulting fluorinated amines of the formula

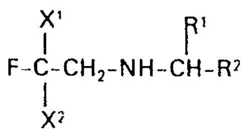

(V)

in which
$X^1$, $X^2$, $R^1$ and $R^2$ have the abovementioned meaning,
are reacted with halogenoacetic acid chlorides of the formula

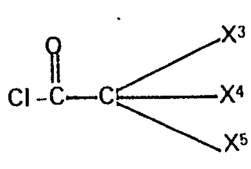

(VI)

in which
$X^3$, $X^4$ and $X^5$ have the abovementioned meaning, if appropriate in the presence of an acid-binding agent and if appropriate in the presence of a diluent, at temperatures between 0°C and 80°C.

**Revendications**

1. Azométhines fluorées de formule

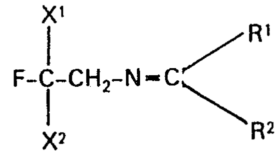

(I)

dans laquelle
$X^1$ représente l'hydrogène, le fluor ou le chlore,
$X^2$ représente l'hydrogène, le fluor ou le chlore,
$R^1$ représente l'hydrogène, un groupe méthyle, éthyle, n-propyle ou n-butyle et
$R^2$ représente l'hydrogène, un groupe méthyle ou éthyle.

2. Procédé de préparation des azométhines fluorées de formule I selon la revendication 1, caractérisé en ce que l'on fait réagir des amines fluorées de formule

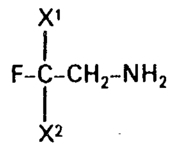

(II)

dans laquelle
$X^1$ et $X^2$ ont les significations indiquées dans la revendication 1, avec des composés carbonylés de formule

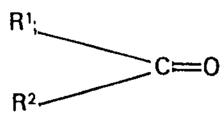

(III)

dans laquelle
$R^1$ et $R^2$ ont les significations indiquées dans la revendication 1, éventuellement en présence d'un diluant.

3. Utilisation des azométhines fluorées de formule I selon la revendication 1, en tant que produits intermédiaires de la synthèse d'halogénoacétamides fluorés de formule

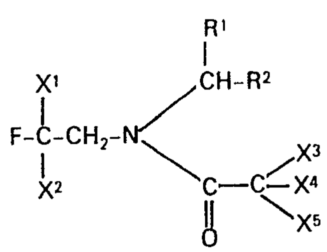

(IV)

dans laquelle
$X^1$, $X^2$, $R^1$ et $R^2$ ont les significations indiquées dans la revendication 1,
$X^3$ représente l'hydrogène, le fluor ou le chlore,
$X^4$ représente l'hydrogène, le fluor ou le chlore et
$X^5$ représente le fluor ou le chlore,
caractérisée en ce que l'on hydrogène les azométhines fluorées de formule

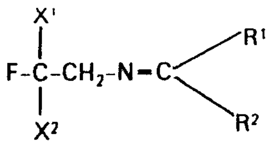

(I)

dans laquelle $X^1$, $X^2$, $R^1$ et $R^2$ ont les significations indiquées ci-dessus, par l'hydrogène sous une pression de 3 à 15 bars en présence d'un catalyseur et en présence d'un diluant à des températures de 0 à 60°C, ce qui donne les amines fluorées de formule

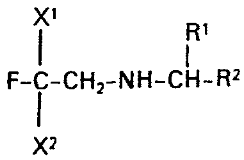

(V)

dans laquelle, $X^1$, $X^2$, $R^1$ et $R^2$ ont les significations indiquées ci-dessus, qu'on fait réagir avec des chlorures d'acides halogénoacétiques de formule

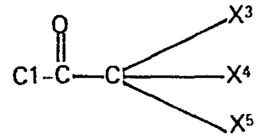

(VI)

dans laquelle $X^3$, $X^4$ et $X^5$ ont les significations indiquées ci-dessus, éventuellement en présence d'un agent fixant les acides et éventuellement en présence d'un diluant, à des températures de 0 à 80°C.